Europäisches Patentamt

European Patent Office

Office européen des brevets

(19)

(11) Veröffentlichungsnummer: **0 246 350**
**A1**

(12) **EUROPÄISCHE PATENTANMELDUNG**

(21) Anmeldenummer: **86107026.6**

(22) Anmeldetag: **23.05.86**

(51) Int. Cl.⁴: **A61B 17/39**

(43) Veröffentlichungstag der Anmeldung:
**25.11.87 Patentblatt 87/48**

(84) Benannte Vertragsstaaten:
**DE GB IT NL**

(71) Anmelder: **Erbe Elektromedizin GmbH.**
**Ebertstrasse 35**
**D-7400 Tübingen(DE)**

(72) Erfinder: **Farin, Günter**
**Kapellenweg 15**
**D-7400 Tübingen-Hirschau(DE)**
Erfinder: **Fischer, Klaus**
**Immengasse 1**
**D-7270 Nagold-Emmingen(DE)**

(74) Vertreter: **Endlich, Fritz, Dipl.-Phys.**
**Postfach 1326 Blumenstrasse 8**
**D-8034 Germering(DE)**

(54) **Koagulationselektrode.**

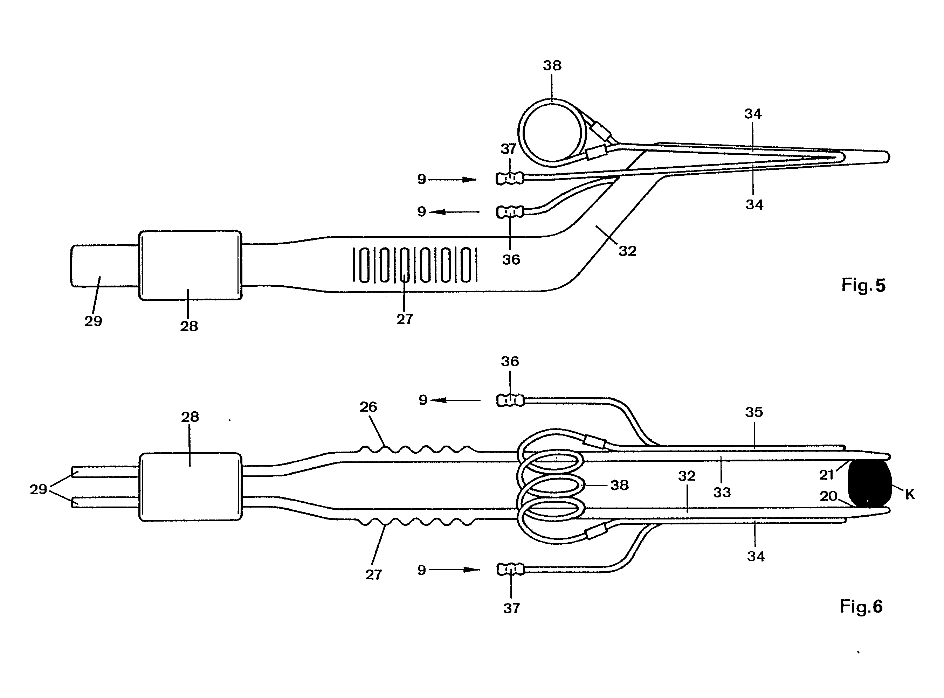

(57) Es werden Koagulationselektroden zum thermischen Koagulieren biologischen Gewebes mittels hochfrequenten elektrischen Wechselstroms beschreiben, bei denen der an die Kontaktfläche der Koagulationselektrode angrenzende Bereich als Wärmesenke mit einem so großen Wert der Wärmekapazität ausgebildet ist und auf eine derart niedrige Temperatur gekühlt wird, daß die Kontaktfläche während der für mindestens einen Koagualtionsvorgang erforderlichen Zeitspanne eine Temperatur innerhalb eines Temperaturbereichs hat, dessen oberste Temperaturgrenze unterhalb der Temperatur des Operationsraums und des unterste Grenze oberhalb der Gefriertemperatur von Wasser liegt. Die Wärmesenke kann eine Kühleinrichtung mit einer Zuleitung und einer Ableitung für flüssiges oder gasförmiges Kühlmittel enthalten. Gemäß einem die Anwendung der Erfindung auf eine bipolare Koagulationspinzette betreffenden Ausführungsbeispiel (Fig. 6) führen für die Zuleitung und die Ableitung des Kühlmittels zu dem Bereich der beiden Kontaktflächen (20,21) metallische Rohrleitungen (34,35), die über eine Verbindungsleitung (38) in Reihe angeschlossen sind, welche Verbindungsleitung durch einen Schlauch aus elektrisch nicht leitendem Kunststoff gebildet ist.

Fig 6

## Koagulationselektrode

Die Erfindung betrifft eine monopolare oder bipolare Koagulationselektrode zum thermischen Koagulieren biologischen Gewebes mittels hochfrequenten elektrischen Wechselstroms, die angrenzend an ihre Koagulations-Kontaktfläche aus einem Material mit guter thermischer Leitfähigkeit besteht, um ein Ankleben des Koagulats an die Kontaktfläche zu vermeiden.

Die thermische Koagulation biologischer Gewebe mittels hochfrequenten elektrischen Wechselstroms wird seit vielen Jahren in verschiedenen Fachbereichen der Chirurgie angewendet, um beispielsweise erkranktes Gewebe zu devitalisieren oder perforierte Blutgefäße zu schließen. Letzteres wird Blutstillung genannt. Für diese Koagulation sind inzwischen viele verschiedene Koagulationselektroden entwickelt, gebaut und verwendet worden.

Durch die Erfindung wird angestrebt, bei derartigen Koagulationselektroden im Hinblick auf das unerwünschte Ankleben des Koagulats an die Kontaktfläche und im Hinblick auf eine gewünschte Eindringtiefe Verbesserungen zu erzielen. Ein seit vielen Jahren bekanntes Problem bei der Hochfrequenz-Koagulation besteht darin, daß das Koagulat während des Koagulationsvorgangs an die Oberfläche der Koagulationselektrode anklebt. Wird die Koagulationselektrode nach dem Koagulationsvorgang vom Gewebe entfernt, so wird infolge des Klebeeffekts ein Teil oder das ganze Koagulat vom Gewebe abgerissen und bleibt an der Oberfläche der Koagulationselektrode haften. Dies führt insbesondere bei der Anwendung der Hochfrequenz-Koagulation zur Blutstillung dazu, daß das durch die Koagulation verschlossene Blutgfäß beim Entfernen der Koagulationselektrode wieder aufgerissen wird. Besonders problematisch ist diese Komplikation in der Neurochirurgie, bei endoskopischen Operationen in der Speiseröhre, dem Magen und dem Dickdarm, sowie in der Mikrochirurgie.

Bei der Tiefenkoagulation besteht eine Schwierigkeit darin, daß bei Verwendung bekannter Koagulationselektroden die maximal erreichbare Koagulatinstiefe physikalisch begrenzt ist, weil die endogene Erwärmung des Gewebes mit dem Abstand von der Kontaktfläche überproportional abnimmt. So erreicht das Gewebe in nächster Nähe zur Kontaktfläche der Koagulationselektrode bereits die Siedetemperatur von Wasser, bevor das Gewebe in etwas größerem Abstand von der Kontaktfläche de Koagulationselektrode die erforderliche Koagulationstemperatur erreicht.

Aus der DE-PS 20 06 126 ist bereits ein Hochfrequenzchirurgieinstrument mit zwei gegeneinander isolierten Backen bekannt, bei welchem der Klebeeffekt dadurch verhindert werden soll, daß die Ränder der Klemmflächen mit einem derart großen Abrundugnsradius abgerundet sind, daß durch hohe Stromdichten an den Rändern bedingte lokale Überhitzungen des Gewebes vermieden werden, und daß mindestens die Klemmflächen aus einem Werkstoff bestehen, dessen thermische udn elektrische Leitfähigkeit besser als diejenige von Stahl ist. Als geeignete Werkstoffe werden u.a. Silber. Gold, Silber-Kupfer, Silber-Palladium, Silber-Cadmiumoxid oder Silbe-Nickel vorgeschlagen. In der Praxis hat sich jedoch gezeigt. daß damit eine allenfalls geringe Verbesserung hinsichtlich des Klebeeffekts erzielbar ist.

Auch in der US-PS 44 92 231 wird zur Vermeidung des Klebeeffekts für eine bipolare Koagulationspinzette vorgeschlagen, die Arme aus einem Material mit guter thermischer Leitfähigkeit herzustellen.

In der GB-PS 21 44 671 wird eine bipolare Koagulationspinzette beschrieben. bei der über ein Schlauchsystem, welches zwischen den beiden Armen dieser Pinzette angeordnet ist, Flüssigkeit auf die Koagulationsstelle appliziert wird, um den Klebeeffekt zu vermeiden. Problematisch erscheint hierbei, daß die verwendete Flüssigkeit sowohl steril als auch gewebeverträglich sein muß. Nach der Koagulation muß die Flüssigkeit außerdem wieder vom Oeprationsfeld abgesaugt oder abgetupft werden.

Es ist auch eine Einrichtung für die Hochfrequenzkoagulation bekannt, bei der Thermosensoren in die Koagulationselektroden installiert sind. Sobald die Temperatur der Elektroden einen bestimmten Wert, beispielsweise 80°C erreicht, wird der hochfrequente Koagulationsstrom manuell oder automatisch abgeschaltet, um den Klebeeffekt zu reduzieren.

Es ist deshalb Aufgabe der Erfindung, eine Koagulationselektrode der eingangs genannten Art derart zu verbessern, daß einerseits das Ankleben des Koagulats an die Kontaktfläche möglichst weitgehend verringert werden kann und andererseits die Möglichkeit erzielt werden kann, daß gewünschtenfalls eine größere Eindringtiefe bei der Tiefenkoagulation erreichbar ist.

Diese Aufgabe wird bei einer Koagulationselektrode der eingangs genannten Art erfindungsgemäß dadurch gelöst, daß der an die Kontaktfläche angrenzende Bereich der Koagulationselektrode als Wärmesenke mit einem so großen Wert der Wärmekapazität ausgebildet ist und auf eine derart niedrige Tem peratur gekühlt wird, daß die Kontaktfläche während der für mindestens einen Koagulationsvorgang erforderlichen Zeitspanne eine Temperatur innerhalb eines Temperaturbereichs hat, dessen oberste Temperaturgrenze unterhalb der Temperatur des Operationsraums und dessen unterste Temperaturgrenze oberhalb der Gefriertemperatur von Wasser liegt. Vorteilhafte Weiterbildungen sind Gegenstand der Unteransprüche.

Die Erfindung beruht insbesondere auf der Erkenntnis, daß für den Klebeeffekt und für die Eindringtiefe bei Tiefenkoagulationen folgende reproduzierbare Effekte von Bedeutung sind:

1. Während der Koagulationsvorgänge werden die Koagulationselektroden indirekt durch das Koagulat erwärmt. Hierbei steigt die Temperatur der Koagulationselektroden, ausgehend von der Temperatur des umgebenden Raums, z.B. 20°C auf mehr oder weniger hohe Temperaturen an. Die nach jedem Koagulationsvorgang ·auf der Oberfläche der Koagulationselektroden verbleibenden Gewebeflüssigkeiten und Gewebereste trocknen je nach der Temperatur der Elektroden mehr oder weniger schnell von der Oberfläche der Elektrode ab und hinterlassen die nicht verdampfenden Bestandteile, die sich nach undnach als immer dicker werdende Schicht auf der Oberfläche der Elektroden aufbauen. Nach mehreren Koagulationen ist diese Schicht schließlich so dick, daß effiziente Koagulationen nicht mehr möglich sind und das Koagulat mehr und mehr an die Koagulationselektroden anklebt.

2. Steigt die Temperatur des Koagulats während der Koagulation über ca. 80°C an, so entstehen im Koagulat klebrige Bestandteile. Es wird angenommen, daß hierbei Kollagen thermisch in Glukose umgewandelt wird In ähnlicher Weise wird Knochenleim hergestellt. Trocknet das Koagulat, in welchem durch thermische Umwandlung klebrige Bestandteile entstanden sind, während des Koagulationsvorgangs an der Grenzschicht zwischen Koagulationselektrode und Koagulat aus, so neigt das Koagulat besonders stark zum Ankleben an die Koagulationselektrode.
Die beiden oben genannten Ursachen für den Klebeeffekt können auch kombiniert wirken.

3. Daß bei einer ausreichenden Kühlung der Koagulationselektrode das Gewebe in der Nähe der Kontaktfläche der Koagulationselektrode nicht koaguliert wird sondern nur tiefere Gewebeschichten, ist medizinisch nicht relevant, da dieses nicht koagulierte Gewebe durch die koagulierte Gewebeschicht vom Stopfwechsel des Körpers isoliert wird.

Bei einer koagulationselektrode gemäß der Erfindung wird deshalb die Temperatur der Koagulationselektrode stets unterhalb der umgebenden Raumtemperatur gehalten. Hierdurch wird bezüglich des ersten oben aufgeführten Effekts erreicht, daß die auf der Oberfläche der Koagulationselektroden verbleibenden Gewebeflüssigkeiten nicht abtrocknen, sondern im Gegenteil die Luftfeuchtigkeit auf der kalten Oberfläche der Koagulationselektrode kondensiert und so die Oberfläche stets feucht bleibt und· keine vertrocknete Gewebeschicht auf der Oberfläche der Koagulationselektroden entsteht. Bezüglich des zweiten oben aufgeführten Effekts wird durch die gegenüber der Raumtemperatur tiefere Temperatur der Oberfläche der Koagulationselektroden während der Koagulationsvorgänge in der Grenzschicht zwischen der Oberfläche der Koagulationselektroden und dem Koagulat keine thermische Umwandlung der verschiedenen Gewebebestandteile in Klebstoffe wie beispielsweise Kollagene in Glukose verursacht, so daß die Koagulationselektrode ohne Klebeeffekt leicht vom Koagulat gelöst werden kann.

Je kälter die Oberfläche der Koagulationselektroden während der Koagulationsvorgänge und während der Koagulationspausen bleibt, desto geringer ist der Klebeeffekt. Allerdings darf die Temperatur nicht den Gefrierpunkt der Gewebeflüssigkeit erreichen, da die Koagulationselektrode dann am Gewebe festfriert. Ideal für die Temperatur der Oberfläche der Koagulationselektroden ist der Temperaturbereich zwischen dem Gefrierpunkt von Wasser und der Raumtemperatur des Operationsraums. Um dies zu erreichen, muß die vom Koagulat in die Koagulationselektroden hineinfließende Wärme stets so schnell abgeleitet werden, daß die Oberfläche der Koagulationselektroden stets kälter ist als dieRaumtemperatur des Operationsraums. Zur Erreichung dieser Bedingung ist allgemein eine Wärmesenke, deren Temperatur unterhalb der Raumtemperatur des Operationsraums liegt, sowie eine Wärmeleitung erforderlich, durch welche die aus dem Koagulat und aus der Luft des Operationsraums in die Koagulationselektrode hineinfließende Wärme ausreichend - schnell in die Wärmesenke abgeleitet werden kann. Dies kann beispielsweise dadurch erfolgen, daß nahe der Kontaktstelle der Koagulationselektrode mit dem Koagulat eine ausreichende Wärmekapazität angebracht ist, welche in den Koagulationspausen in sterilem Eiswasser abgekühlt wird und von der Temperatur des Eiswassers ausgehend bis zum Erreichen der Raumtemperatur des Operationsraums soviel Wärmemenge aufnehmen kann, daß bei bestimmungsgemäßer Anwendung der Koagulationselektrode ausreichend lange koaguliert werden kann, bevor die Oberfliche der Koagulationselektrode an den Kontaktstellen zum Koagulat die Raumtemperatur des Operationsraums erreicht. Dies kann beispielsweise auch in der Weise erfolgen, daß die Koagulationselektrode möglichst nahe der Kontaktstelle mit dem Koagulat mit Eiswasser durchströmt wird. Dies kann beispielsweise auch dadurch erfolgen, daß innerhalb der Koagulationselektrode möglichst nahe der Kontaktstelle zum Koagulat komprimierte Gase zur Expansion gebracht werden, wodurch der Koagulationselektrode Wärme entzogen wird.

Bei der praktischen Erprobung der erfindungsgemäßen Koagulationselektrode für die Hochfrequenzkoagulation wurde ein weiterer für die Koagulationstechnik wichtiger Vorteil beobachtet. Bekanntlich entsteht in der Grenzschicht zwischen der Koagulationselektrode und dem Koagulat, wo die elektrische Stromdichte am größten ist und die Erwärmung des Gewebes am schnellsten erfolgt, beim Erreichen der Siedetemperatur der Gewebeflüssigkeiten eine Dampfschicht, welche die Koagulationselektrode vom Gewebe elektrisch isoliert, wodurch der Koagulationsvorgang unterbrochen wird. Je höher die elektrische Stromdichte ist, desto schneller entsteht die elektrisch isolierende Dampfschicht und desto schneller wird der Koagulationsvorgang unterbrochen. Hierdurch wird die gewünschte Koagulationstiefe bzw. das gewünschte Volumen des Koagulats oft nicht erreicht. Wird jedoch die erfindungsgemäße Koagulationselektrode verwendet, bei welcher deren Oberfläche so gekühlt wird, daß deren Temperatur möglichst nahe dem Gefrierpunkt von Wasser liegt, so wird das Gewebe an der Grenzschicht zur Koagutionselektrode nicht auf die Siedetemperatur der Gewebeflüssigkeit aufgeheizt. Außerdem kondensiert der Wasserdampf, der in der Grenzschicht entsteht, sehr schnell an der kalten Oberfläche der Koagulationselektroden. Hierdurch wird die elektrisch isolierende Dampfschicht verhindert und die Koagulationsvorgänge nicht vorzeitig unterbrochen.

Ein weiterer Vorteil der erfindungsgemäßen Koagulationselektrode ist , daß deren Oberfläche auch nach mehrmaliger Koagulation sauber bleibt. Hieraus resultiert indirekt der weitere Vorteil, daß während der Koagulation der Übergangswiderstand der Grenzschicht ausreichend klein bleibt, wodurch derart hohe elektrische Feldstärken verhindert werden, bei welchen elektrische Lichtbogen oder Funken entstehen, die die Oberfläche der Koagulationselektroden erodieren.

Ein besonderer Vorteil der Erfindung ergibt sich ferner bei der Durchführung von Tiefenkoagulationen, weil bei derartigen Koagulationen ein größerer Bereich der Eindringtiefe zur Verfügung steht, weil das Gewebe in nächster Nähe zur Kontaktfläche der Koagulationselektrode nicht die Siedetemperatur von Wasser wie bei bekannten Koagulationselektroden erreicht, bevor das Gewebe in etwas größerem Abstand von der Kontaktfläche der Koagulationselektrode die erforderliche Koagulationstemperatur erreicht hat. Dank der Kühlung der kontaktnahen Gewebeschicht können deshalb tiefere Gewebeschichten koaguliert werden, bevor die Temperatur des kontaktnahen Gewebes die Siedetemperatur von Wasser erreicht. Daß das Gewebe in der Nähe der Kontaktfläche hierbei nicht koaguliert wird, sondern nur tiefere Gewebeschichten, ist medizinisch nicht relevant, da das nicht koagulierte Gewebe durch die koagulierte Gewebeschicht vom Stoffwechsel des Körpers isoliert wird.

Anhand der Zeichnung soll die Erfindung beispielsweise näher erläutert werden. Es zeigen:

Fig. 1 ein Ausführungsbeispiel einer monopolaren Koagulationselektrode mit einer zusätzlichen Wärmekapazität, in perspektivischer Darstellung;

Fig. 2 die Koagulationselektrode in Fig. 1 in geschnittener Darstellung;

Fig. 3 ein Ausführungsbeispiel einer bipolaren Koagulationselektrode mit zusätzlichen Wärmekapazitäten;

Fig. 4 ein abgewandeltes Ausführungsbeispiel einer monopolaren Kogulationselektrode, welche mit flüssigem Kühlmittel gekühlt wird;

Fig. 5 und 6 ein weiteres abgewandeltes Ausführungsbeispiel einer bipolaren Koagulationselektrode, welche mit einem flüssigen Kühlmittel gekühlt wird;

Fig. 7 ein weiteres abgewandeltes Ausführungsbeispiel einer monopolaren Koagulationselektrode, welche durch expandierendes Gas gekühlt wird;

Fig. 8 ein weiteres abgewandeltes Ausführungsbeispiel einer bipolaren Koagulationselektrode, welche durch expandierendes Gas gekühlt wird; und

Fig. 9 bis 11 grafische Darstellungen zur Erläuterung der Wirkungsweise bekannter Koagulationselektroden im Vergleich zu Koagulationselektroden gemäß der Erfindung.

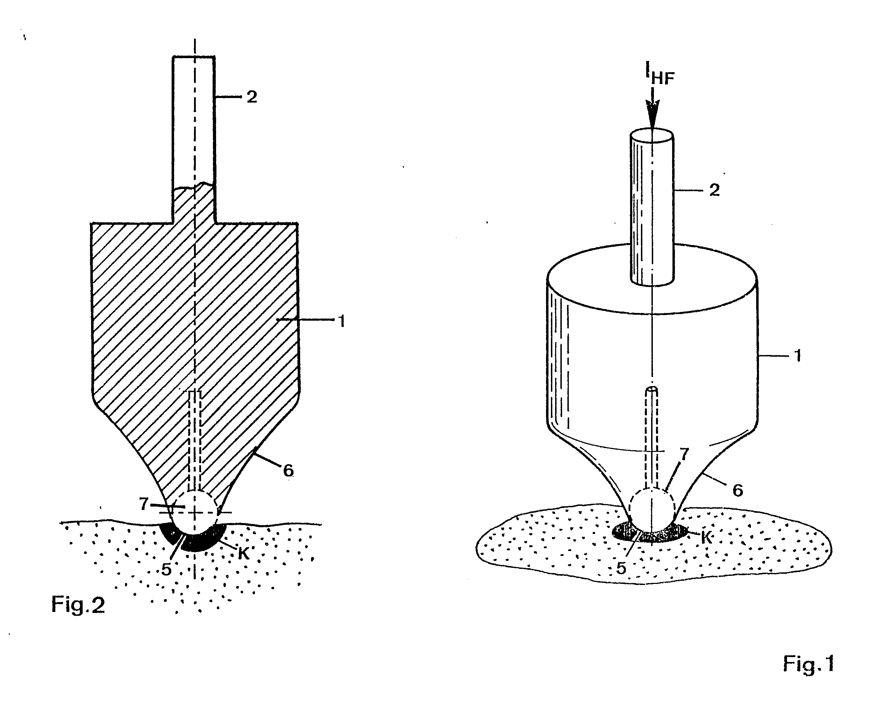

In Fig. 1 ist ein Ausführungsbeispiel einer monopolaren Koagulationselektrode dargestellt, bei welcher im Vergleich zu einer bekannten monopolaren Koagulationselektrode 7, die in Fig. 1 punktiert eingezeichnet ist, nahe der Kontaktfläche 5 der Koagualtionselektrode mit dem Koagulat K eine zusätzliche Wärmekapazität in Form eines Kühlkörpers 1 so angeordnet ist, daß die Wärme, die während der Koagulation in der Grenzschicht zwischen der Kontaktfläche 5 und dem Koagulat K entsteht, schnell in die vorher unter die Temperatur des Operationsraums abgekühlte zusätzliche Wärmekapazität abfließen kann. Der Übergang 6 zwischen der Kontaktfläche 5 und dem Kühlkörper 1 soll einerseits möglichst kurz sein, andererseits die Sicht auf das Koagulat K möglichst nicht verdecken. Die zusätzliche Wärmekapazität kann beispielsweise aus Metall bestehen, wobei das Metall eine möglichst gute Wärmeleitfähigkeit und eine große spezifische Wärmekapazität haben soll. Bei bekannten monopolaren Koagulationselektroden, beispielsweise bei der in Fig. 1 punktiert angedeuteten Kugelelektrode 7, ist die Wärmekapazität nahe der Kontaktfläche 5 praktisch vernachlässigbar klein im Vergleich zur Größe der Kontaktfläche, so daß deren Wärmekapazität auch dann nicht ausreicht, die Temperatur der Kontaktfläche während eines einzigen Koagulationsvorgangs zuverlässig unterhalb der Temperatur des Operationsraums zu halten, wenn deren Wärmekapazität, welche lediglich durch die Masse der Kugel gebildet wird, vor dem Koagulationsvorgang beispielsweise auf 0°C abgekühlt wird. Noch ungünstiger verhalten sich diesbezüglich bekannte monopolare Koagualtionselektroden, bei welchen die Kontaktfläche nicht durch eine Kugel, sondern durch eine Metallplatte gebildet wird, welche bei diesen als Plattenelektroden bekannten Koagultionselektroden eine Kontaktfläche von etwa 25 bis 500 mm² und eine Dicke von etwa 0,5 bis 2 mm haben.

In Fig. 2 ist die monopolare Koagulationselektrode in Fig. 1 im Schnitt dargestellt, wobei wiederum eine konventionelle bekannte Kugelelektrode 7 punktiert in das Schnittbild eingezeichnet ist, um deutlich zu machen, was mit zusätzlicher Wärmekapazität bei der erfindungsgemäßen Koagulationselektrode gemeint ist. Der Kühlkörper 1 soll so dimensioniert sein, daß die Temperatur der Kontaktfläche 5 nach mindestens einem Koagulationsvorgang noch unterhalb der Temperatur des Operationsraums bleibt, wenn diese Wärmekapazität vor dem Koagulationsvorgang unter die Temperatur des Operationsraums, jedoch nicht tiefer als 0°C gekühlt wird.

Der hochfrequente Wechselstrom $I_{HF}$ fließt bei der erfindungsgemäßen Koagulationselektrode den gleichen Weg wie bei bekannten Koagulationselektroden.

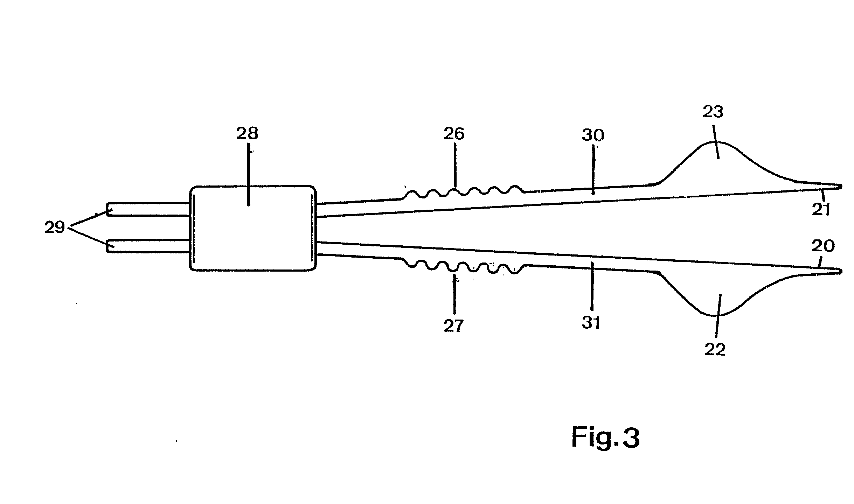

Fig. 3 zeigt schematisch ein Ausführungsbeispiel einer bipolaren Koagulationselektrode in Form einer bipolaren Koagulationspinzette, wobei analog zu dem Ausführungsbeispiel in den Fig. 1 oder 2 möglichst nahe den Kontaktflächen 20 und 21 je ein Kühlkörper 22 und 23 ausgebildet ist, welche vor der Ausführung der Koagulation in sterilem Kältemittel, beispielsweise in Eiswasser gekühlt werden, und deren Wärmekapazität so bemessen werden kann, daß die Temperatur der Kontaktflächen 20 und 21 auch nach mehreren, beispielsweise zehn Koagulationen unterhalb der Temperatur des Operationsraums bleibt. Im Ausführungsbeispiel der Fig. 3 sind die beiden Kühlkörper 22 und 23 so geformt, daß die Sicht des Operateurs auf die Kontaktflächen 20 und 21 und auf das Gewebe, welches während der Koagulation zwischen diesen beiden Kontaktflächen gehalten wird, nicht behindert wird.

Um zu verhindern, daß die beiden zusätzlichen Wärmekapazitäten 22 und 23 von der Hand des Operateurs aufgewärmt werden bzw. daß die abgekühlte bipolare Koagualtionselektrode dem Operateur unangenehm kalt erscheint, ist es vorteilhaft, die Branchen 30 und 31 und insbesondere die Griffbereiche 26 und 27 der bipolaren Koagulationselektrode mit einem Kunststoff zu beschichten, der eine ausreichende Wärmeisolation bietet. Diese Wärmeisolation kann außerdem die elektrische Isolation darstellen, welche bei bipolaren Koagulationselektroden, insbesondere bei bipolaren Koagulationspinzetten erforderlich ist.

Eine weitere Ausgestaltung der Erfindung besteht darin, die Koagulationselektrode kontinuierlich so zu kühlen, daß die Temperatur der Kontaktfläche 5, welche die Koagulationselektrode mit dem zu koagulierenden Gewebebzw. mit dem Koagulat bildet, automatisch im Temperaturbereich zwischen der Temperatur des Operationsraums und dem Gefrierpunkt von Wasser bleibt. Dies wird beispielsweise erreicht, indem die Koagulationselektrode möglichst nahe der Kontaktfläche 5 mittels eines flüssigen Kühlmittels, beispielsweise mittels Eiswasser gekühlt wird.

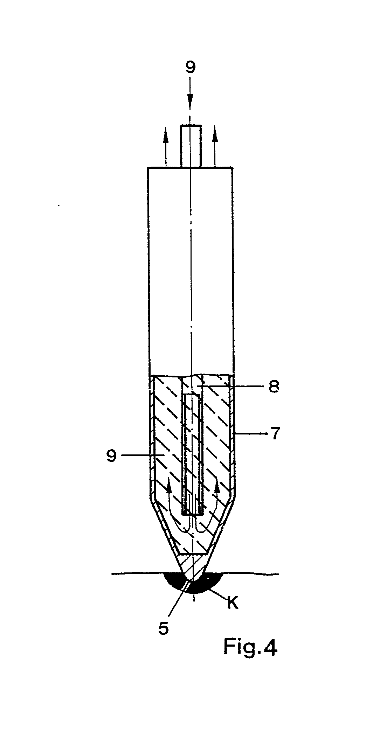

Fig. 4 zeigt schematisch ein Ausführungsbeispiel einer mittels flüssigem Kühlmittel 9 gekühlten monopolaren Koagulationselektrode. Das flüssige Kühlmittel 9, dessen Temperatur im o.g. Temperaturbereich liegt, wird beispielsweise mittels einer elektrisch betriebenen Rollenpumpe durch ein Zuleitungsrohr 8 innerhalb der monopolaren Koagulationselektrode bis nahe an die Kontaktfläche 5 getrieben, von wo aus es, ebenfalls innerhalb dieser monopolaren Koagulationselektrode, zurückfließt.

Hierbei nimmt es die aus dem Koagulat K durch die Kontaktfläche 5 in die Koagulationselektrode hineinfließende Wärme mit.

Die Fig. 5 und 6 zeigen schematisch ein Ausführungsbeispiel einer mittels flüssigen Kühlmittels 9 gekühlten bipolaren Koagulationselektrode in Form einer bipolaren Koagulationspinzette. Das flüssige Kühlmittel 9, dessen Temperatur ebenfalls im o.g. Temperaturbereich liegt, wird durch je ein Rohr 34 bzw. 35, welche mit gutem Wärmekontakt auf den vorderen Bereich der Branchen 32 bzw. 33 geklebt oder gelötet sind, geleitet, und kühlt so die vorderen Bereiche der Branchen 32 und 33 und damit die Kontaktflächen 20 und 21, so daß die Temepratur der Kontaktflächen 20 und 21 stets unterhalb der Temperatur des Operationsraums von beispielsweise 25°C bleibt.

Die Rohre 34 und 35 können sowohl aus Metall als auch aus Kunststoff bestehen. Metallrohre haben den Vorteil, daß sie auf die Branchen 32 und 33 der bipolaren Pinzette aufgelötet werden können und somit sehr gute Voraussetzungen für den Wärmeübergang von den Branchen 32 bzw. 33 in die Rohre 34 bzw. 35 bieten. Metallrohre haben jedoch den Nachteil, insbesondere wenn sie direkt auf die Branchen der bipolaren Koagulationspinzette gelötet werden, daß hierdurch ein elektrischer Kurzschluß zwischen den beiden Branchen der bipolaren Pinzette entsteht. Bei monopolaren Koagulationspinzetten würde dieser Kurzschluß durch die Verbindung 38 der beiden Rohre 34 und 35 nicht stören, weilbei monopolaren Koagulationspinzetten die beiden Branchen bestimmungsgemäß elektrisch leitfähig miteinander verbunden sind. Zur Vermeidung dieses elektrischen Kurzschlusses bei bipolaren Koagulationspinzetten erfolgt die Verbindung 38 der beiden Rohre 34 und 35 durch einen elektrisch nicht leitfähigen Kunststoffschlauch. Hierbei ist es zwar unvermeidlich, daßdurch das innerhalb des Kunststoffschlauchs der Verbindung 38 befindliche Wasser elektrischer Strom fließen kann, in der Praxis stört dies jedoch dann nicht, wenn diese Verbindung 38 eine ausreichende Länge, beispielsweise 5 cm hat und der Querschnitt nicht unnötig groß ist, beispielsweise nicht größer als 10 mm². Infolge der relativ großen Länge und des relativ kleinen Querschnitts der Verbindung 38 im Vergleich zur Länge und zum Querschnitt des Koagulats K ist der elektrische Widerstand der Verbindung 38 wesentlich größer als der des zu koagulierenden Gewebes, so daß der relativ geringe Strom, der über die Verbindung 38 parallel zum Strom durch das zu koagulierende Gewebe fließt, praktisch vernachlässigbar klein bleibt.

Bei Verwendung von Kunststoffrohren für die Rohre 34 und 35 wird ein elektrischer Kurzschluß oder ein elektrischer Bypass zwischen den beiden Branchen 32 und 33 zwar vermieden, allerdings ist der Wärmeübergangswiderstand bei Kunststoffrohren größer als bei Metallrohren.

Das flüssige Kühlmittel, beispielsweise Eiswasser, wird bei der in den Fig. 5 und 6 dargestellten bipolaren Koagulationselektrode in den Anschlußstutzen 36 eingeleitet und aus dem Anschlußstutzen 37 abgeleitet, wobei die Richtung auch getauscht werden kann.

Ähnlich wie bei dem in Fig. 3 dargestellten Ausführungsbeispiel ist es auch bei dem in den Fig. 5 und 6 dargestellten Ausführungsbeispiel zweckmäßig, die Wärmeleitfähigkeit der Branchen im Griffbereich 26 und 27 möglichst klein zu gestalten, damit einerseits die Wärme der Hand des Operateurs nicht das Kühlsystem belastet und andererseits der Operateur nicht ein unangenehmes Kältegefühl durch diese Koagulationselektrode erfährt. Dieses Problem kann, wie beim Ausführungsbeispiel in Fig. 3 beschrieben, gelöst werden.

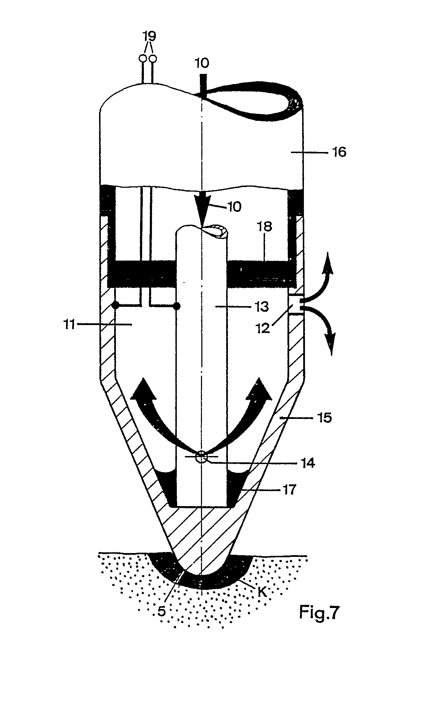

Eine weitere Möglichkeit zur Kühlung der Koagulationselektrode ist in Fig. 7 dargestellt. Innerhalb dieser Koagulationselektrode wird durch eine Leitung 13 komprimiertes Gas 10, beispielsweise Preßluft, $CO_2$ oder $N_2O$, in die Koagulationselektrode eingeleitet. Die Leitung 13 endet nahe der Kontaktlfäche 5 in einer kleinen Düse bzw. in ein kleines Loch 14, bei welchem das komprimierte Gas ausströmt, wobei es expandiert und hierbei abkühlt. Dieses Prinzip ist bereits in der Kryochirurgie bekannt, wo nach diesem Prinzip Gewebe gefroren wird. Bei der hier dargestellten Koagulationselektrode darf die Temperatur der Kontaktfläche 5 jedoch nicht so weit abkühlen, daß an ihrer Oberfläche Wasser zu Eis gefriert. Aus diesem Grund muß bei dieser Koagulationselektrode die Temperatur kontrolliert werden. Dies kann beispielsweise durch ein Thermoelement erfolgen, welches möglichst nahe der Kontaktstelle 5 innerhalb der Koagulationselektrode angeordnet ist. In der Koagulationselektrode in Fig.7 ist das Thermoelement dadurch gebildet, daß die Leitung 13 aus einem Metall besteht, welches mit dem Metall der Außenwand 15 eine möglichst hohe thermoelektrische Spannung bildet. Die thermoelektrische Spannung zwischen der Leitung 13 und der Außenwand 15 ist ein Maß für die Temperatur der Lötstelle 17 nahe der Kontaktfläche 5. Die thermoelektrische Spannung kann an den Klemmen 19 abgenommen werden.

Ist die Koagulationselektrode ausreichend groß, so kann auch ein separates Thermoelement oder ein temperaturabhängiger elektrischer Widerstand innerhalb der Koagulationselektrode nahe der Kontaktfläche 5 angeordnet werden.

Mittels der thermoelektrischen Spannung kann über ein elektronisch gesteuertes Gasventil der Gasstrom so gesteuert werden, daß die Temperatur der Lötstelle 17 und damit der Kontaktstelle 5 dicht oberhalb der Gefrier temperatur von Wasser, beispielswëise 5°C, bleibt.

Das expandierte Gas kann entweder aus einer Öffnung 12 aus der Expansionskammer 11 direkt ausströmen oder mit Rücksicht auf die Sterilität des Operationsfelds durch einen Schlauch in nicht sterile Bereiche abgeleitet werden.

Um zu verhindern, daß einerseits Wärme aus der Hand des Operateurs das Kühlsystem belastet und andererseits der Elektrodengriff, mit welchem der Operateur diese Koagulationselektrode hält, unangenehm kalt wird, ist nur die Wand 15 der Expansionskammer 11 aus Metall mit guter Wärmeleitfähigkeit gebaut. Die Abschlußwand 18 sowie die Verlängerung 16 der Halterung dieser Koagulationselektrode sind dagegen aus einem Material mit möglichst geringer Wärmeleitfähigkeit, beispielsweise Kunststoff, hergestellt.

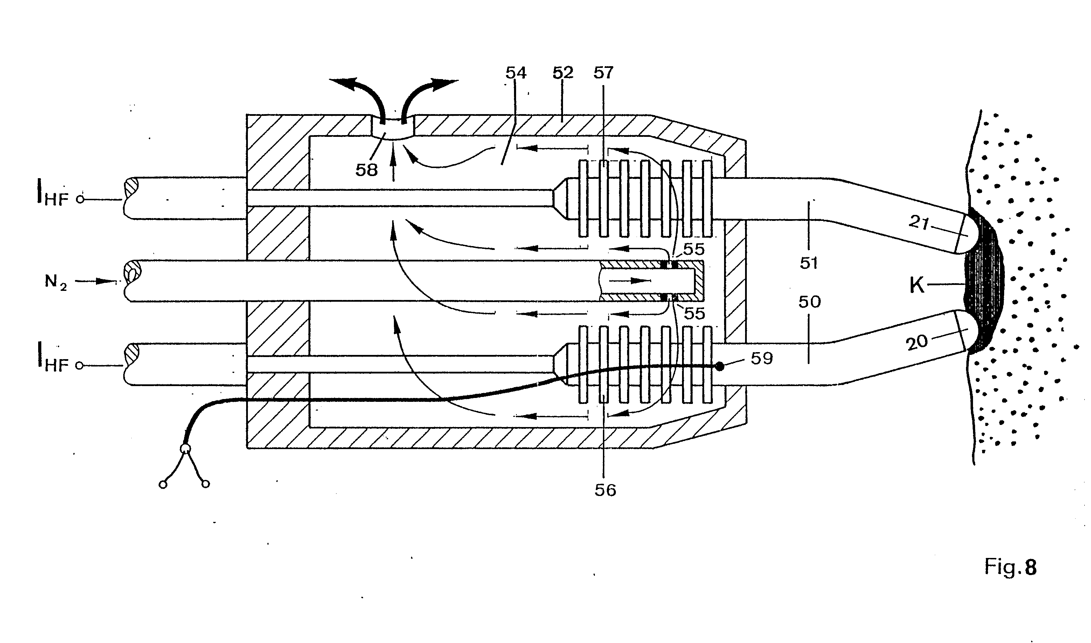

In Fig. 8 ist schematisch ein Ausführungsbeispiel einer bipolaren Koagulationselektrode dargestellt, welche analog dem Ausführungsbeispiel der Fig. 7 mittels expandierenden Gases gekühlt wird. Die beiden Pole 50 und 51 dieser bipolaren Elektroden werden möglichst nahe der Kontaktflächen 20,21 durch eine Expansionskammer 54 geführt, innerhalb welcher komprimiertes Gas, beispielsweise N₂, CO₂ oder N₂0 aus der Düse 55 ausströmt und expandiert, wodurch dieses. Gas abkühlen und somit die beiden Pole 50 und 51 kühlen kann. Zur Verbesserung des Wärmeübergangs von den Polen 50 bzw. 51 in das kalte Gas innerhalb der Expansionskammer 54 sind beide Pole 50 und 51 innerhalb der Expansionskammer 54 mit Kühlflächen 56 bzw. 57 ausgestattet. Das expandierte Gas kann entweder aus einer Öffnung 58 aus der Expansionskammer 54 direkt ausströmen oder mit Rücksicht auf die Sterilität des Operationsfelds durch einen Schlauch in nicht sterile Bereiche abgeleitet werden. Um zu verhindern, daß die Kontaktflächen 20 und 21 tiefer als die Schmelztemperatur von Wasser abgekühlt werden, ist innerhalb der Expansionskammer 54 ein Temperatursensor 59, beispielsweise an einer der beiden Pole 50 oder 51 angeordnet, welcher die Temperatur der identisch ausgestalteten Pole 50 und 51 überwacht. Der Gasstrom durch die Düse 55 wird beispielsweise mit einem elektromagnetischen Ventil, dessen Steuerung von der Temperaturmessung durch den Temperatursensor 59 abhängig ist, so gesteuert, daß die Temperatur an der Meßstelle des Temperatursensors 59, und damit hinreichend genau an den Kontaktflächen 20 und 21 automatisch geregelt wird. Die Pole 50 und 51 sind so gestaltet, daß die Wärmeleitfähigkeit zwischen den Kühlflächen 56 bzw. 57 und den Kontaktflächen 20 bzw. 21 möglichst groß ist, zwischen den gleichen Kontaktflächen und dem Anschluß der elektrischen Leitungen für den hochfrequenten Wechselstrom I_HF jedoch möglichst klein ist. Die Fig. 8 zeigt schematisch nur die erfindungsrelevanten Details dieses Ausführungsbeispiels. Je nach Verwendungszweck kann diese bipolare Elektrode mit geeigneten Griffen ausgestattet werden und/oder die Pole 50 und 51 optimal geformt werden. Die Wand 52 der Expansionskammer 54 besteht aus elektrisch und thermisch isolierendem Material.

Die Form der beispielsweise in den Fig. 1 bis 8 dargestellten Koagulationselektroden kann dem jeweiligen Verwendungszweck angepaßt werden, wobei dies prinzipiell sowohl für monopolare als auch für bipolare Koagulationselektroden gilt.

Besonders vorteilhaft können diese gekühlten Koagulationselektroden zum Blutstillen, beispielsweise in der Speiseröhre, im Magen oder im Dickdarm, verwendet werden, weil im Vergleich zu konventionellen, nicht gekühlten Koagulationselektroden das Gewebe in der Grenzschicht zwischen der jeweiligen Kontaktfläche der Koagulationselektrode und dem Koagulat das Gewebe nicht koaguliert und insbesondere auch nicht austrocknet. Die erfindungsgemäß gekühlten Koagulationselektroden können nach der Vollendung einer Blutstillung wieder vom Gewebe entfernt werden, ohne daß das Blutgefäß erneut aufgerissen wird. Vorteilhaft sind Koagulationseinrichtungen entsprechend dieser Erifndung auch für Tiefenkoagulationen. Bei konventionellen, nicht gekühlten Koagulationselektroden ist die maximal erreichbare Koagulationstiefe physikalisch begrenzt, weil die endogene Erwärmung des Gewebes mit dem Abstand von der Kontaktfläche überproportional abnimmt. So erreicht das Gewebe in nächster Nähe zur Kontaktfläche der Koagulationselektrode bereits die Siedetemperatur von Wasser, bevor das Gewebe in etwas größerem Abstand von der Kontaktfläche der Koagulationselektrode die erforderliche Koagulationstemperatur erreicht. Dank der Kühlung der kontaktnahen Gewebeschicht durch die erfindungsgemäße Koagulationseinrichtung können tiefere Gewebebereiche koaguliert werden, bevor die Temperatur des kontaktnahen Gewebes die Siedetemperatur von Wasser erreicht. Daß das Gewebe in der Nähe der Kontaktfläche der Koagulationselektrode hierbei nicht koaguliert wird, sondern nur tiefere Schichten, ist medizinisch nicht relevant, da dieses nicht koagulierte Gewebe durch die koagulierte Zone vom Stoffwechsel des Körpers isoliert wird.

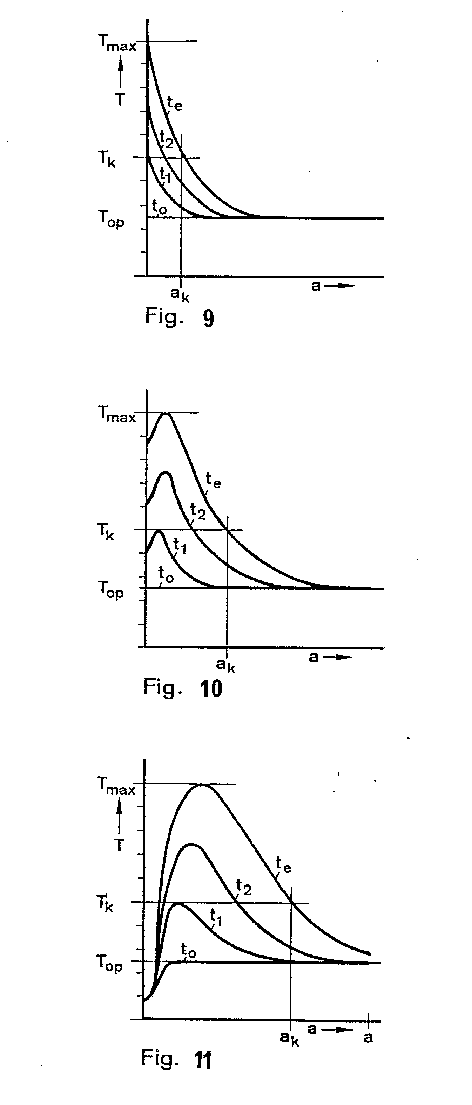

Anhand der Fig. 9 bis 11 wird dieser Vorteil dargestellt. In allen drei Diagrammen ist die Temperatur T des Gewebes über dem Abstand a von der Kontakfläche der Koagulationselektrode dargestellt, wobei die Koagulationsdauer t als Parameter dargestellt ist. Die Kurve t₀ zeigt in allen drei Diagrammen das Temperaturprofil am Beginn des Koagulationsvorgangs. Die Kurve tₑ zeigt in allen drei Diagrammen das Temperaturprofil am Ende des Koagulationsvorgangs. Die Kurven t₁ und t₂ zeigen beispielsweise Temperaturprofile zwischen Beginn und Ende eines Koagulationsvorgangs.

In Fig. 9 sind schematisch die Temperaturprofile t₀ bis tₑ bei Verwendung einer bekannten Koagulationselektorde ohne Kühlung der Kontaktfläche und mit geringer Wärmeleitfähigkeit des Materials, aus welchem diese Koagulationselektrode besteht, dargestellt. Bei dieser Koagulationselektrode wird die Kontaktfläche so schnell aufgeheizt, daß deren Temperatur fast unverzögert der Temperatur des Koagulats folgt. Hierbei erreicht die Kontaktfläche bereits während eines Koagulationsvorgangs Temperaturen oberhalb 50°C, wodurch es sehr schnell zum Anstieg des elektrischen Widerstands in der Gewebeschicht nahe der Kontaktfläche der Koagulationselektrode infolge Koagulation, Dampfbildung und Austrocknung dieser Gewebeschicht kommt, was wiederum sehr schnell die Intensität des elektrischen Stroms $I_{HF}$ durch die Koagulationselektrode verringert und dadurch den Koagulationsvorgang derart behindert, daß nur eine relativ geringe Koagulationstiefe $a_K$ entstehen kann. Die Koagulationstiefe $a_K$ meint hier den maximalen Abstand von der Kontaktfläch der Koagulationselektrode, in welchem die Koagulationstemperatur $T_K$ noch erreicht wird.

In Fig. 10 sind schematisch die Temperaturprofile $t_0$ bis $t_e$ bei Verwendung einer Koagulationselektrode entsprechend DE-PS 20 06 125 oder US-PS 44 92 231 dargestellt. Bei derartigen Koagulationselektroden wird das Gewebe nahe der Kontaktfläche gekühlt, wenn und solange die Kontaktfläche kälter ist als das sie berührende Gewebe. Die Ausgangstemperatur $T_0$ am Anfang des ersten Koagulationsvorgangs, was dem Zeitpunkt $t_0$ entspricht, ist gleich der Raumtemperatur des Operationsraums. Während des Koagulationsvorgangs fließt Wärme aus dem Gewebe bzw. aus dem Koagulat in die Koagulationselektrode hinein, wodurch deren Temperatur, ausgehend von der Raumtemperatur des Operationsraums, mehr oder weniger schnell ansteigt. Bei derartigen Koagulationselektroden kann die Temperatur der Kontaktfläche der Koagulationselektrode bereits während des erstmaligen Koagulationsvorgangs sehr hoch ansteigen. Werden mehrere Koagulationen in kurzer Zeitfolge nacheinander durchgeführt, was in der chirurgischen Praxis oft vorkommt, so erreichen diese Koagualtionselektroden trotz der guten Wärme leitfähigkeit des Materials, aus welchem sie bestehen, relativ schnell hohe Temperaturen, denn die Wärmekapzität dieser Koagulationselektroden ist relativ klein.

In Fig. 11 sind schematisch die Temperaturprofile $t_0$ bis $t_e$ bei Verwendung einer Koagulationselektrode entsprechend der Erfindung dargestellt.Da die Koagulationselektrode auf eine Temperatur unterhalb der Raumtemperatur gekühlt wird, beispielsweise auf 5°C, bleibt die Kontaktfläche dieser Koagulationselektrode während der gesamten Koagulationsdauer unterhalb der Raumtemperatur $T_{OP}$ des Operationsraums. Hierdurch kann im Grenzbereich zwischen der Kontaktfläche der Koagulationselektrode und dem Gewebe kein Wasserdampf entstehen, der den HF-Strom behindert und den Koagualtionsvorgang frühzeitig beendet. Der maximale Temperaturanstieg verlagert sich in tiefere Gewebeschichten.

Der Koagulationsvorgang sollte stets beendet werden, bevor die Temperatur innerhalb des Gewebes den Siedepunkt der Gewebeflüssigkeit erreicht, weil andernfalls das Koagulat infolge des dann innerhalb des Koagulats entstehenden Dampfdrucks explosionsartig zerplatzt. Unter Beachtung dieses Kriteriums ist aus den Diagrammen in Fig. 9 bis 11 deutlich erkennbar, daß die maximal erreichbare Koagulationstiefe $a_K$ mit der erfindungsgemäßen Koagulationseinrichtung größer ist als mit bekannten Koagulationselektroden.

**Ansprüche**

1. Monopolare oder bipolare Koagulationselektrode zum thermischen Koagulieren biologischen Gewebes mittels hochfrequenten elektrischen Wechselstroms, die angrenzend an ihre Koagulations-Kontaktfläche aus einem Material mit guter thermischer Leitfähigkeit besteht, **dadurch gekennzeichnet,** daß der an die Kontaktfläche (5;20,21) angrenzende Bereich der Koagulationselektrode als Wärmesenke mit einem so großen Wert der Wärmekapazität ausgebildet ist und auf eine derart niedrige Temperatur gekühlt wird, daß die Kontaktfläche (5;20,21) wähend der für mindestens einen Koagulationsvorgang erforderlichen Zeitspanne eine Temperatur innerhalb eines Temperaturbereichs hat, dessen oberste Temperaturgrenze unterhalb der Temperatur des Operationsraums und dessen unterste Temperaturgrenze oberhalb der Gefriertemperatur von Wasser liegt.

2. Koagulationselektrode nach Anspruch 1, **dadurch gekennzeichnet,** daß die Wärmesenke durch einen Kühlkörper (1:22,23) aus Metall gebildet ist, der vor einem Koagulationsvorgang über seine Oberfläche durch eine Kühlmittel abgekühlt wird.

3. Koagulationselektrode nach Anspruch 1, **dadurch gekennzeichnet,** daß die Wärmesenke eine Kühleinrichtung mit einer Zuleitung (8;13;34) und einer Ableitung für Kühlmittel enthält.

4. Koagulationselektrode nach Anspruch 3, **dadurch gekennzeichnet,** daß die Zuleitung (8;13;34) möglichst nahe zu einer an die Kontaktfläche (5;20;21) angrenzenden Innenwandfläche der Koagulationselektrode einmündet.

5. Koagulationselektrode nach Anspruch 3 oder 4, **dadurch gekennzeichnet,** daß die Mündung der Zuleitung (13) durch eine Düse (14) gebildet ist und daß das zugeführte Kühlmittel ein komprimiertes Gas ist.

6. Koagulationselektrode nach einem der Ansprüche 3 bis 5, **dadurch gekennzeichnet,** daß in der Nähe der Kontaktfläche (5;20;21) ein Temperatursensor (13,17,15;59) angeordnet ist, der einer elektronischen Schaltung für eine derartige Steuerun der Kühlmittelströmung zugeordnet ist, daß die Temperatur der Kontaktfläch (5;20;21) inner halb des genannten Temperaturbereichs liegt.

7. Koagulationselektrode nach Anspruch 6, **dadurch gekennzeichnet,** daß als Temperaturmeßfühler ein Thermoelement vorgesehen ist, das durch eine metallische Zuleitung (13), eine aus einem anderen Metall bestehende, die Kühleinrichtung umgebende Außenwand (15), sowie durch eine dazwischen vorgesehene Lötstelle (17) gebildet ist.

8. Koagulationselektrode nach einem der vorhergehenden Ansprüche, **dadurch gekennzeichnet,** daß in dem Bereich der beiden Branchenenden (30,31) einer Koagulationspinzette jeweils eine Wärmesenke ausgebildit ist, und daß vorzugsweise die beiden Griffbereiche (26,27) der Koagulationspinzette mit wärmeisolierendem Kunststoff beschichtet sind.

9. Koagualtionselektrode nach Anspruch 8, **dadurch gekennzeichnet,** daß für die Zuleitung und Ableitung des Kühlmittels zu dem Bereich der beiden Kontaktflächen (20,21) Rohrleitungen (34,35) aus Metall und/oder Kunststoff vorgesehen sind, die über eine Verbindungsleitung (38) in Reihe angeschlossen sind.

10. Bipolare Kogulationselektrode nach Anspruch 9, **dadurch gekennzeichnet,** daß die Verbindungsleitung (38) durch einen Schlauch aus elektrisch nicht leitendem Kunststoff gebildet ist.

11. Bipolare Koagulationselektrode nach einem der Ansprüche 1, 3, 5 oder 6, **dadurch gekennzeichnet,** daß als Kühleinrichtung eine von einer isolierenden Außenwand (52) umgebene Expansionskammer (54) vorgesehen ist, aus der zwei Pole (50,51) vorragen, an deren äußeren Enden die beiden Kontaktflächen (20,21) ausgebildet sind, und daß das mit mindestens einer Düse (55) versehene Austrittsende der Zuleitung für das komprimierte Gas zwischen den in die Expansionkammer (54) vorragenden, vorzugsweise jeweils mit einer Kühlfläche (51, 57) versehenen inneren Enden der Pole (50,51) angeordnet ist.

Fig.2

Fig.1

0 246 350

Fig.3

Fig.4

Fig.5

Fig.6

Fig.7

Fig.8

0 246 350

Fig. 9

Fig. 10

Fig. 11

Europäisches Patentamt

# EUROPÄISCHER RECHERCHENBERICHT

Nummer der Anmeldung

EP 86 10 7026

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | US-A-4 074 718 (CH. MORRISON) * Figuren 1-7,13,14,27; Spalte 3, Zeilen 50-61; Spalte 5, Zeilen 19-27; Spalte 6, Zeilen 22-39; Spalte 7, Zeilen 1-36; Spalte 8, Zeilen 23-26; Spalte 14, Zeilen 45-59,64-68 * | 1-4 | A 61 B 17/39 |
| A | | 5 | |
| | --- | | |
| A | DE-A-3 215 832 (J. DOSS) * Figuren; Seite 12, Absatz 3 - Seite 13, Zeile 8 * | 1-4 | |
| | --- | | |
| A | DE-A-2 656 278 (K. SEMM) | 5 | |
| | --- | | RECHERCHIERTE SACHGEBIETE (Int. Cl.4) |
| A | GB-A-2 002 236 (ERBE GmbH) | | A 61 B |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 15-01-1987 | VEREECKE A. |